# EUROPEAN PATENT APPLICATION

(11) **EP 4 670 659 A2**
(43) Date of publication of application: **31.12.2025**
(21) Application number: 23932179.7
(22) Date of filing: 11.04.2023
(51) Int. Cl.: A61B 18/12, A61B 18/14, A61N 1/36, A61B 18/00

(54) **ELECTRODE DEVICE FOR BLOCKING OR CONTROLLING NERVES IN BODY**

(30) Priority: 04.04.2023 KR 20230043866
(71) Applicant: Deepqure Inc., Seoul 06243 (KR)
(72) Inventor: HONG, Suk Ki, Seoul 06243 (KR); YOON, Young Il, Seoul 06243 (KR); BACH, Du Jin, Seoul 06243 (KR); KIM, Kee Wan, Seoul 06243 (KR); PARK, Chan Ho, Seoul 06243 (KR); PARK, Jong Sun, Seoul 06243 (KR)
(74) Representative: Manitz Finsterwald Patent- und Rechtsanwaltspartnerschaft mbB
(86) International application number: PCT/KR2023/004833
(87) International publication number: WO 2024/210244

(57) **Abstract**

An electrode apparatus for blocking or controlling nerves in a body includes: a main body including a shaft; an electrode unit formed to be drawn out from one end of the shaft and configured to denervate or modulate at least a part of nerves on a tube in the body; an electrode guide including a plurality of joint units and a wire connecting the plurality of joint units to each other and configured to guide the electrode unit; a sensor unit formed in a predetermined portion of the electrode unit and configured to measure a temperature of the tube in the body; and a signal processor configured to convert an analog signal value measured by the sensor unit into a digital signal value and transmit the converted digital signal value to an RF energy generator.

## Description

### TECHNICAL FIELD

The present disclosure relates to an electrode apparatus for blocking or controlling nerves in body.

### BACKGROUND

Denervation is a surgical procedure intended to control an abnormally overactive autonomic nervous system by damaging specific nerves. For example, renal denervation can treat hypertension and heart diseases by damaging renal sympathetic nerves directed to the kidney, and pulmonary denervation can treat lung diseases by damaging parasympathetic nerves directed to the lung.

When the surgical procedure is performed as described above, it is important to accurately transmit electrical impulses required for the surgical procedure to nerves which are a procedure target. Specifically, in order to effectively denervate or modulate the nerves on a tube in a body, it is important to accurately transfer RF energy required for the surgical procedure depending on a state of the tube with distributed nerves (*e.g*., a temperature of the tube, etc.).

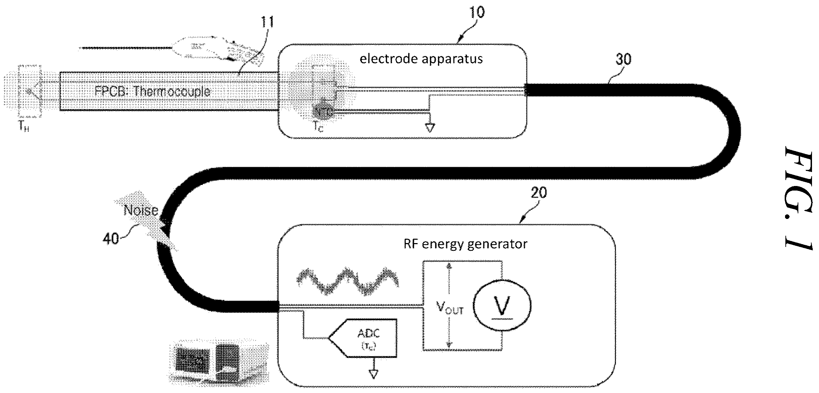

**FIG. 1** illustrates a process of processing a signal of a temperature value of a tube in the body measured by an electrode apparatus according to the prior art.

Referring to **FIG. 1****,** temperature data of the tube in the body measured by a thermocouple 11 in an electrode apparatus 10 are processed in an RF energy generator 20 through a cable 30.

For example, an analog signal measured by the electrode apparatus 10 is transmitted to the RF energy generator 20 through the cable 30, and the RF energy generator 20 converts the received analog signal into a digital signal. Further, the RF energy generator 20 outputs RF energy to the electrode apparatus 10 based on the converted digital signal.

Herein, while the analog signal measured by the electrode apparatus 10 is transmitted to the RF energy generator 20 through the cable 30, it may be exposed to noise 40 in the cable 30 and the accuracy of the measured analog signal, *i.e.*, the measured temperature, may be decreased. In particular, the electrode apparatus 10 measures the temperature of the tube in the body, which is a procedure target, through the thermocouple 11, and is greatly affected even by small noise 40 since a voltage difference generated in the thermocouple 11 is as low as several µV per 1°C.

### DISCLOSURE OF THE INVENTION

### PROBLEMS TO BE SOLVED BY THE INVENTION

The present disclosure is conceived to provide an electrode apparatus which measures a temperature of a procedure target and accurately transmits the measured temperature to an RF energy generator, and, thus, the RF energy generator accurately outputs RF energy required for denervation.

Also, the present disclosure is conceived to provide an electrode apparatus capable of accurately transmitting information about a temperature of a procedure target measured by a thermocouple to an RF energy generator.

Further, the present disclosure is conceived to provide an electrode apparatus which is improved to reduce an error in measurement signals between the electrode apparatus and an RF energy generator connected to each other through a cable.

The problems to be solved by the present disclosure are not limited to the above-described problems. There may be other problems to be solved by the present disclosure.

### MEANS FOR SOLVING THE PROBLEMS

According to an aspect of the present disclosure, an electrode apparatus for blocking or controlling nerves in a body includes: a main body including a shaft; an electrode unit formed to be drawn out from one end of the shaft and configured to denervate or modulate at least a part of nerves on a tube in the body; an electrode guide including a plurality of joint units and a wire connecting the plurality of joint units to each other and configured to guide the electrode unit; a sensor unit formed in a predetermined portion of the electrode unit and configured to measure a temperature of the tube in the body; and a signal processor configured to convert an analog signal value measured by the sensor unit into a digital signal value and transmit the converted digital signal value to an RF energy generator.

The electrode unit may include: a base layer; a plurality of electrode layers disposed on the base layer; and a top layer disposed to overlap a part of the electrode layers with the plurality of electrode layers interposed therebetween.

The sensor unit may be a thermocouple composed of a first metal and a second metal, and the thermocouple may include a cold junction disposed on the base layer.

The above-described aspects are provided by way of illustration only and should not be construed as limiting the present disclosure. In addition to the above-described embodiments, there may be additional embodiments described in the accompanying drawings and the detailed description.

### EFFECTS OF THE INVENTION

An electrode apparatus according to the present disclosure can accurately transmit information about a temperature of a procedure target measured by a thermocouple to an RF energy generator. Thus, it is possible to reduce an error in measurement signals between the electrode apparatus and the RF energy generator connected to each other through a cable and also possible to precisely perform a surgical procedure.

### BRIEF DESCRIPTION OF THE DRAWINGS

**FIG. 1** illustrates a process of processing a signal of a temperature value of a tube in the body measured by an electrode apparatus according to the prior art.
**FIG. 2** is a side view of an electrode apparatus according to an embodiment of the present disclosure.
**FIG. 3** illustrates a state where an electrode guide illustrated in **FIG. 2** guides and locates an electrode unit to enclose a blood vessel.
**FIG. 4** is a plan view of a part of the electrode unit illustrated in **FIG. 2****.**
**FIG. 5** illustrates a signal processor disposed inside a main body illustrated in **FIG. 2****.**
**FIG. 6A and 6B** illustrate a sensor unit of an electrode unit in an area A illustrated in **FIG. 5****.**
**FIG. 7** illustrates components disposed inside a shaft in an area A illustrated in **FIG. 3****.**
**FIG. 8** is an exploded perspective view of a part of a joint unit illustrated in **FIG. 3****.**
**FIG. 9** is a cross-sectional view of a driving unit disposed inside the main body illustrated in **FIG. 2****.**
**FIG. 10A to FIG. 10C** illustrate an operation process of the electrode guide according to an embodiment of the present disclosure.

### BEST MODE FOR CARRYING OUT THE INVENTION

Hereinafter, embodiments of the present disclosure will be described in detail with reference to the accompanying drawings to be readily implemented by a person with ordinary skill in the art to which the present invention belongs. However, it is to be noted that the present disclosure is not limited to the example embodiments but can be embodied in various other ways. In the drawings, parts irrelevant to the description are omitted in order to clearly explain the present disclosure, and like reference numerals denote like parts through the whole document.

Through the whole document, the term "connected to" or "coupled to" that is used to designate a connection or coupling of one element to another element includes both a case that an element is "directly connected or coupled to" another element and a case that an element is "electronically connected or coupled to" another element via still another element. Further, it is to be understood that the term "comprises or includes" and/or "comprising or including" used in the document means that one or more other components, steps, operation and/or existence or addition of elements are not excluded in addition to the described components, steps, operation and/or elements unless context dictates otherwise and is not intended to preclude the possibility that one or more other features, numbers, steps, operations, components, parts, or combinations thereof may exist or may be added. Through the whole document, the term "on" that is used to designate a position of one element with respect to another element includes both a case that the one element is adjacent to the other element and a case that any other element exists between these two elements.

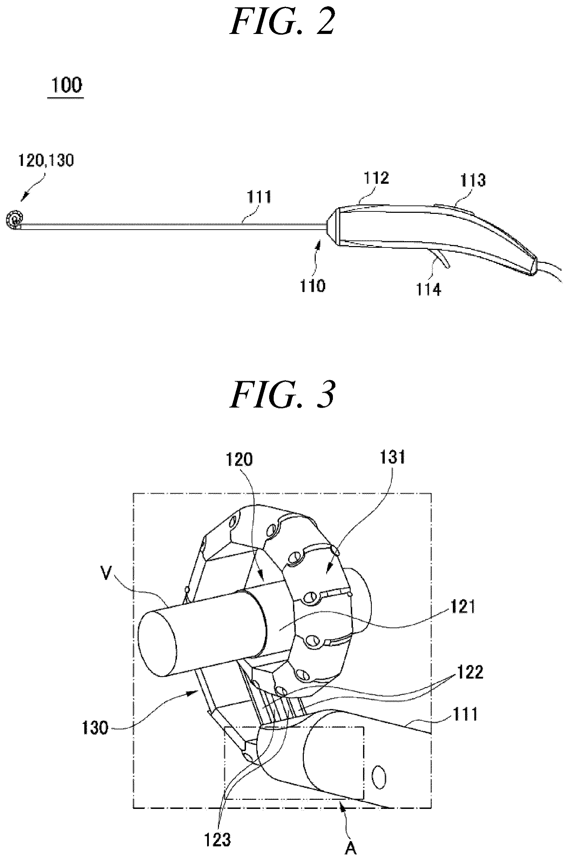

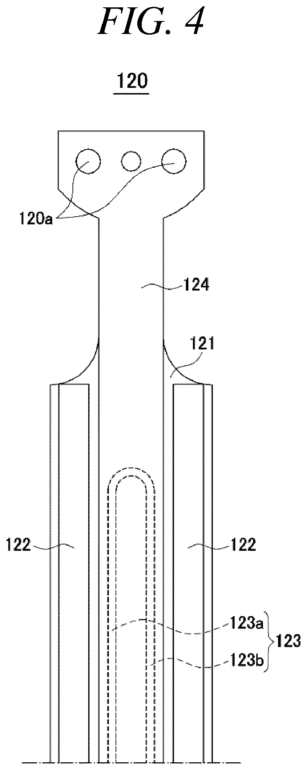

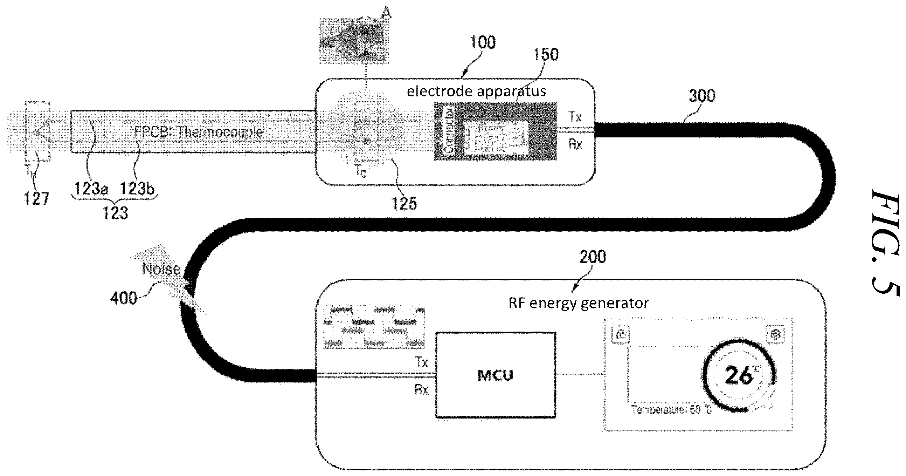

**FIG. 2** is a side view of an electrode apparatus 100 according to an embodiment of the present disclosure, and **FIG. 3** illustrates a state where an electrode guide 130 illustrated in **FIG. 2** guides and locates an electrode unit 120 to enclose a blood vessel. **FIG. 4** is a plan view of a part of the electrode unit illustrated in **FIG. 2****,** and **FIG. 5** illustrates a signal processor disposed inside a main body illustrated in **FIG. 2****.**

Referring to **FIG. 2****,** the electrode apparatus 100 according to an embodiment of the present disclosure includes a main body 110, the electrode unit 120, and the electrode guide 130. The main body 110 may include a shaft 111 extending in one direction, a grip portion 112 connected to the shaft 111 so as to be gripped by an operator, a guide manipulation unit 113 formed on the grip portion 112 so as to manipulate an operation of the electrode guide 130, and an electrode manipulation unit 114 formed on the grip portion 112 so as to manipulate energy transfer to the electrode unit 120. The components for driving and controlling the electrode unit 120 and the electrode guide 130 may be located inside the main body 110.

The electrode unit 120 is formed to be drawn out from one end of the shaft 111 and configured to denervate or modulate at least a part of nerves distributed on a tissue including a tube in the body depending on manipulation by the operator. The electrode unit 120 is accommodated inside the shaft 111 and when the electrode apparatus 100 of the present disclosure operates, the electrode unit 120 can be drawn out by means of the electrode guide 130 which will be described later.

Referring to **FIG. 3****,** the electrode guide 130 functions to bring the electrode unit 120 into contact with the tube in the body. The electrode guide 130 supports the electrode unit 120 and guides the electrode unit 120 to be in contact with the tube in the body.

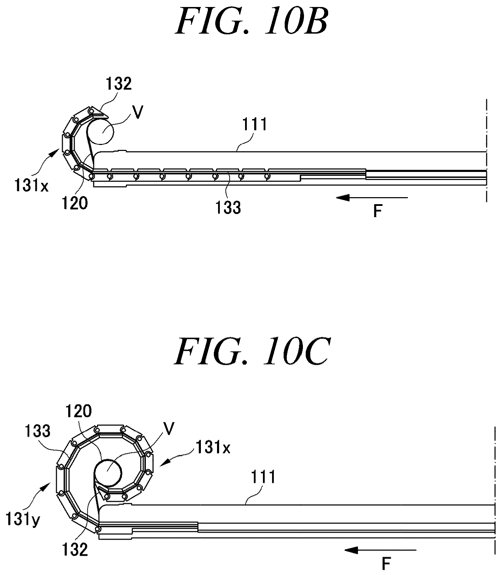

The electrode guide 130 of the present disclosure includes a plurality of joint units 131. The plurality of joint units 131 forms a curved winding path to enclose the circumference of a tube V in the body with the electrode unit 120 interposed therebetween. The state illustrated in **FIG. 3** and **FIG. 10C** may be a state where the plurality of joint units 131 is completely drawn out and disposed along the curved winding path.

Referring to **FIG. 3** and **FIG. 4****,** the electrode unit 120 may include a base layer 121, an electrode layer 122, and a top layer 124. In the electrode apparatus 100 of the present disclosure, an electrode encloses an outer surface of the tube or tube-shaped tissue V in the body and energy can be transferred through the electrode. To this end, the base layer 121 may be formed as a flexible printed circuit board (PCB).

The electrode layer 122 is formed on the base layer 121, and may be composed of two electrodes extending parallel to each other on the base layer 121 in the embodiment illustrated in **FIG.** 3. According to the present embodiment, the base layer 121 and the electrode layer 122 may be configured to extend and enclose the tube in the body or the like in a circumferential direction.

The electrode layer 122 may be made of a material, such as stainless steel or gold, which is harmless to the human body and conducts electricity well in order to block, denervate, control, or modulate the nerves. Also, the electrode layer 122 may transfer various types of energy from an energy source generator. For example, the energy may include radio-frequency (RF) energy, electrical energy, laser energy, ultrasonic energy, high-intensity focused ultrasound energy, cryogenic energy and other heat energy.

Further, the electrode layer 122 may be implemented as a flexible PCB for transferring RF energy, a transducer for transferring ultrasonic energy or a metal electrode for transferring high-voltage energy and thus may transfer energy to damage the nerves.

Furthermore, the electrode unit 120 may include the base layer 121, the electrode layer 122 disposed on the base layer 121, and the top layer 124 disposed to overlap a part of the electrode layer 122 with the electrode layer 122 interposed between the base layer 121 and the top layer 124. A through-hole 120a may be formed in the top layer 124.

Also, the electrode unit 120 may include a sensor unit 123. The sensor unit 123 is formed in a predetermined portion of the electrode unit 120 and configured to measure a temperature of the tube in the body. When neurotomy is performed with the electrode apparatus 100 according to the present disclosure, the sensor unit 123 can monitor a temperature of a treatment site.

The sensor unit 123 may be a thermocouple that measures a temperature by contact with the tube in the body or the like. For example, the sensor unit 123 may be a thermocouple formed on the base layer 121 and may include a first metal 123a and a second metal 123b. For example, the first metal 123a may be copper and the second metal 123b may be a constantan.

Referring to **FIG. 5****,** the sensor unit 123 is a thermocouple composed of the first metal 123a and the second metal 123b, and includes a cold junction (CJ) 125 and a hot junction (HJ) 127. In general, the thermocouple generates an electromotive force, *i.e.*, thermoelectromotive force, by connecting two kinds of metals 123a and 123b having different junction temperatures and allows a current to flow.

Herein, the thermocouple includes points where the first metal 123a transitions to and is connected to the second metal 123b, and these points are referred to as the cold junction 125 and the hot junction 127. The sensor unit 123, *i.e.,* the thermocouple, according to the embodiment illustrated in **FIG.** 5 may be disposed on the base layer 121 and may constitute a cold junction.

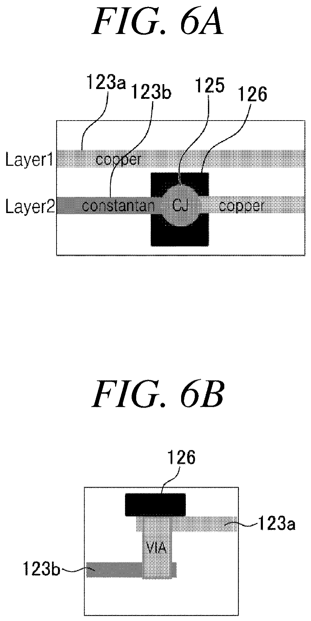

**FIG. 6A and FIG. 6B** illustrate a sensor unit of an electrode unit in an area A illustrated in **FIG. 5****.** Referring to **FIG. 6A****,** a signal processor 150 may insert a temperature sensor 126 into the CJ 125. That is, referring to **FIG. 6B****,** the signal processor 150 may locate the temperature sensor 126 at a portion where the first metal 123a starts and the second metal 123b ends to reduce an error in temperature measurement through the thermocouple.

Therefore, the sensor unit may be disposed in the electrode unit to increase the accuracy in temperature measurement at the cold junction and improve the accuracy of a temperature measured by the thermocouple.

Meanwhile, referring back to **FIG. 5****,** the electrode apparatus 100 according to the present disclosure may directly process a signal of a temperature of the tube in the body measured by the sensor unit 123 inside the main body 110. The electrode apparatus 100 includes the signal processor 150 inside the main body 110 and thus can directly process information about the measured temperature of the tube in the body.

According to the prior art, the signal of the temperature of the tube measured by the electrode apparatus 100 is transmitted to an RF energy generator 200 through a cable 300 and converted into temperature data in the RF energy generator 200, which may cause a decrease in accuracy of information about the temperature of the tube. For example, while an analog signal measured by the sensor unit 123 of the electrode apparatus 100 is transmitted to the RF energy generator 200 through the cable 300, it may be exposed to noise 400 in the cable 300. Thus, the accuracy of the measured temperature value, *i.e.,* the temperature value of the tube in the body, may be decreased.

Therefore, the electrode apparatus 100 according to the present disclosure includes the signal processor 150 inside the main body 110, and the signal processor 150 directly processes a signal of a temperature of a tube measured by the sensor unit 123 and then transmits the signal to the RF energy generator 200. Thus, it is possible to remove the noise 400 in the cable 300 and improve the accuracy of a surgical procedure.

In the embodiment illustrated in **FIG.** 5, the electrode apparatus 100 includes the signal processor 150 inside the main body 110. For example, the electrode apparatus 100 includes a grip portion to be gripped by an operator, *i.e.,* the signal processor 150 disposed inside the main body 110, and thus can convert an analog signal measured by the sensor unit 123 of the electrode unit 120 into temperature data.

The signal processor 150 may convert an analog signal value measured by the sensor unit 123 into a digital signal value. The signal processor 150 may transmit the converted digital signal value to the RF energy generator 200. As such, the electrode apparatus 100 according to the present disclosure may transmit the digital signal value converted by the signal processor 150 to the RF energy generator 200 and thus may suppress distortion or loss of signal.

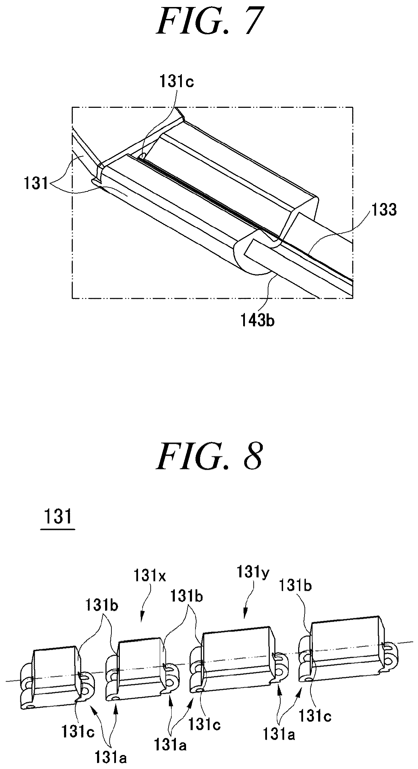

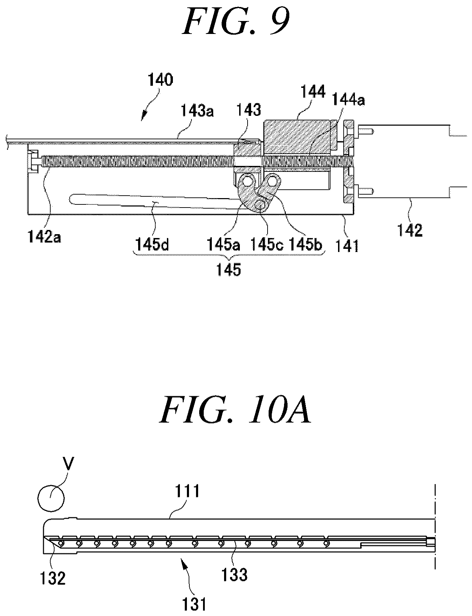

**FIG. 7** illustrates components disposed inside a shaft in an area A illustrated in **FIG. 3****,** and **FIG. 8** is an exploded perspective view of a part of a joint unit illustrated in **FIG. 3****.** **FIG. 9** is a cross-sectional view of a driving unit disposed inside the main body illustrated in **FIG. 2****,** and **FIG. 10A** **to** **FIG. 10C** illustrate an operation process of the electrode guide according to an embodiment of the present disclosure.

Referring to **FIG. 7** and **FIG. 8****,** a wire 133 may be formed to sequentially penetrate the plurality of joint units 131. Each joint unit 131 may include a wire hole 131c in a longitudinal direction to allow penetration of the wire 133. The end of the wire 133 sequentially penetrating the wire holes 131c may be coupled and fixed to a tip joint 132, and the wire 133 can slide with respect to each joint unit 131 in the wire hole 131c in the longitudinal direction. Therefore, the wire 133 can guide the plurality of joint units 131 and the tip joint 132 to be disposed along the winding path and provide a force of pulling the plurality of joint units 131 and the tip joint 132 in a direction to be around the tube V.

The wire 133 may operate to protrude from one end of the shaft 111 together with the plurality of joint units 131. Herein, the wire 133 may be designed to protrude in a smaller amount than the joint units 131. Thus, the wire 133 can provide a force of pulling the plurality of joint units 131 along a curved path.

Referring to **FIG. 8****,** each joint unit 131 may include hinge units 131a and winding support units 131b. The hinge units 131a are configured for rotatable connection to adjacent joints and may be formed on one or both sides of the joint unit 131 in the longitudinal direction in which the joint units 131 are connected parallel to each other. As illustrated in **FIG. 8****,** the hinge unit 131a may have a rotation axis in a direction intersecting the longitudinal direction so as to be connected to the hinge unit 131a of the adjacent joint unit 131. A hinge pin (not illustrated) may be inserted into and fastened to each hinge unit 131a in the direction of the rotation axis.

The winding support units 131b are configured to support the plurality of joint units 131 on the winding path and may be formed on one or both sides of the joint unit 131 in the longitudinal direction to support the adjacent joint unit 131. As illustrated in **FIG. 8****,** the winding support unit 131b may be located adjacent to the hinge unit 131a in an inward direction of the electrode guide 130 (in a direction of winding the joint unit 131). For example, the winding support unit 131b may be formed as a surface having a predetermined angle and area and supported by the adjacent winding support unit 131b in surface contact with each other, and, thus, a wound shape of the electrode guide 130 can be maintained. The winding support unit 131b and the wire hole 131c may be formed at locations spaced apart from a rotation center of the hinge unit 131a in an inward direction toward the tube V in the body.

When the wire 133 is pulled backwards relative to the electrode guide 130 (when a length of the wire 133 drawn out from the shaft 111 is smaller than that of the joint units 131), a tensile force may be applied to the wire 133 in a direction of winding the electrode guide 130. On the other hand, the winding support units 131b may provide a force of supporting the joint units 131 to each other in a direction of suppressing winding of the electrode guide 130. Since the wire 133 and the winding support units 131b form a balanced force in opposite directions, the electrode guide 130 can be fixed on the winding path.

Meanwhile, as illustrated in **FIG. 8****,** the electrode guide 130 may include a first joint group 131x and a second joint group 131y. That is, the plurality of joint units 131 may be divided into the first joint group 131x and the second joint group 131y having different lengths.

Due to a difference in length, the first joint group 131x may form a first radius of curvature and the second joint group 131y may form a second radius of curvature greater than the first radius of curvature. As can be seen from **FIG. 10C****,** the joint units (the first joint group 131x) having a relatively small length may form a smaller radius of curvature and the joint units (the second joint group 131y) having a relatively great length may form a greater radius of curvature.

When the joint units 131 located close to the tip joint 132 form a path having a smaller radius of curvature, a path along which the tip joint 132 enters a space between the tube in the body and the shaft 111 may be formed as shown in **FIG. 10C****.** Also, the electrode guide 130 including the joint units 131 may have an overall spiral shape.

Referring to **FIG. 9****,** a driving unit 140 may include a frame 141, a motor unit 142, a rod block 143, a wire block 144, and a variable connection unit 145. The driving unit 140 drives the joint unit 131 and the wire 133 of the electrode guide 130 to protrude from one end of the shaft and drives the joint unit 131 in conjunction with the wire 133 to have different displacements.

That is, the driving unit 140 may control the joint units 131 and the wire 133 to increase a difference in displacement therebetween in order for the electrode guide 130 to be in a wound state to enclose the circumference of the tube V in the body. Further, the driving unit 140 may control the joint units 131 and the wire 133 to decrease the difference in displacement therebetween in order for the electrode guide 130 to be in a rectilinear state.

For example, the wire 133 may be protruded from one end of the shaft 111 in a smaller amount (length) than the joint units 131 by means of the driving unit 140. The joint units 131 may be pulled in one direction (in a direction to be wound around the tube in the body) by means of the wire 133 as much as a difference in protruding amount and may be protruded while forming a curved winding path. More specifically, whenever the joint units 131 protrude and rotate at a winding angle (for example, 30°) formed by the winding support units 131b, the wire 133 may protrude in a relatively small amount.

As illustrated in **FIG. 9****,** the frame 141 may be provided to be fixed inside the main body and may include a guide slot or guide shaft extending in the forward and backward directions. The motor unit 142 may be connected to the frame 141 and may include a rotation shaft 142a rotatably supported by the frame 141. For example, the motor unit 142 may receive electrical energy to rotate the rotation shaft 142a.

One end of the rod block 143 may be connected to the joint unit 131. The rod block 143 may be moved in the forward and backward directions by means of the motor unit 142. Specifically, the rod block 143 may be moved in the forward and backward directions in engagement with the rotation shaft 142a extending in the forward and backward directions and having a thread thereon. The rod block 143 may include a rod 143a, which is located inside the shaft 111 and extends in one direction (forward and backward directions) and supports the joint units 131, and a corrugated component slidably coupled to the guide slot or guide shaft of the frame 141.

In addition to the above-described rotation shaft 142a and motor unit 142, the driving unit 140 according to the present disclosure may be configured to move the rod block 143 in the forward and backward directions by various linear actuation mechanisms. For example, the driving unit 140 may include a linear actuator of cylinder type including a pneumatic, hydraulic or electric linear actuator, or a piezoelectric or ultrasonic linear actuator.

The wire block 144 may be formed to support the wire 133 and moved in the forward and backward directions in conjunction with the rod block 143. The wire block 144 may include a corrugated component slidably inserted into the guide slot or guide shaft and a sliding hole 144a slidably accommodating the rotation shaft 142a, and may move in the forward and backward directions in parallel to the rod block 143.

The variable connection unit 145 may connect the rod block 143 and the wire block 144 to each other and vary a distance between the rod block 143 and the wire block 144. To this end, the variable connection unit 145 may include a rod link 145a, a wire link 145b, a hinge pin 145c, and a pin slot 145d.

Also, the rod link 145a and the wire link 145b may be rotatably connected to the rod block 143 and the wire block 144, respectively. Further, the rod link 145a and the wire link 145b may be rotatably connected to each other by the hinge pin 145c.

The pin slot 145d is formed to slidably accommodate the hinge pin 145c. Specifically, the pin slot 145d is formed to extend at a predetermined tilt angle with respect to the forward and backward directions. The pin slot 145d may be formed in the frame 141.

According to the present disclosure, the plurality of joint units 131 may be drawn out from the shaft 111 by means of the driving unit 140 and wound in a direction to enclose the tube V. Accordingly, a space where the electrode guide 130 operates can be minimized, and an operation of denervating or modulating nerves can be performed safely and accurately in a narrow space.

Further, since the wire 133 is driven in conjunction with the joint units 131 to have different displacements by means of the driving unit 140, the electrode guide 130 of the electrode apparatus 100 according to the present disclosure can secure precision and repeatability in operation path.

Referring to **FIG. 10A** to **FIG. 10C****,** the electrode guide 130 may further include the tip joint 132 and the wire 133. The tip joint 132 may support the electrode unit 120 and may be coupled to the end of the plurality of joint units 131 connected sequentially to each other. The tip joint 132 may be drawn out from one end of the shaft 111 earlier than the plurality of joint units 131.

As illustrated in **FIG. 10C****,** the tip joint 132 may be located close to the tube V in the body and may have a tapered shape that gradually decreases in width or thickness toward the end in order to suppress interference with the electrode unit 120 or maximize the surface enclosing the tube in the body. The end of the electrode unit 120 may be fastened and fixed to the tip joint 132.

As illustrated in **FIG. 10A** to **FIG. 10C****,** when the plurality of joint units 131 is sequentially drawn out, the plurality of joint units 131 may be sequentially drawn out and moved toward one side and thus may be in the wound state to overall enclose the tube V. However, the electrode guide 130 is spaced apart from an outer circumferential surface of the tube V in the wound state and the electrode unit 120 located inside the wound electrode guide 130 may be in close contact with the outer circumferential surface of the tube V.

Referring to **FIG. 10A** and **FIG. 10B****,** the electrode guide 130 is accommodated together with the electrode unit 120 inside the shaft 111 and may protrude from one end in a forward direction F while forming a curved winding path for surgical procedure.

Referring to **FIG. 10C****,** the electrode guide 130 may be deformed into the wound state to enclose the circumference of the tube V in the body due to the increased difference in displacement between the joint units 131 and the wire 133. Specifically, when the plurality of joint units 131 is sequentially drawn out from the shaft 111, the plurality of joint units 131 may move along the curved winding path due to a difference in displacement from the wire 133 and thus may overall enclose the tube V. Further, the electrode guide 130 is spaced apart from an outer circumferential surface of the tube V and the electrode unit 120 located inside the wound electrode guide 130 may be in close contact with the outer circumferential surface of the tube V.

Accordingly, a space where the electrode guide 130 operates can be minimized, and an operation of denervating or modulating nerves can be performed safely and accurately in a narrow space.

The above description of the present disclosure is provided for the purpose of illustration, and it would be understood by a person with ordinary skill in the art to which the present disclosure belongs that various changes and modifications may be made without changing technical conception and essential features of the present disclosure. Thus, it is clear that the above-described examples are illustrative in all aspects and do not limit the present disclosure. For example, each component described to be of a single type can be implemented in a distributed manner, likewise, components described to be distributed can be implemented in a combined manner.

The scope of the present disclosure is defined by the following claims rather than by the detailed description of the embodiment, and it should be understood that all modifications and embodiments conceived from the meaning and scope of the claims and their equivalents are included in the scope of the present disclosure.

## Claims

1. An electrode apparatus for blocking or controlling nerves in a body, comprising:
a main body including a shaft;
an electrode unit formed to be drawn out from one end of the shaft and configured to denervate or modulate at least a part of nerves on a tube in the body;
an electrode guide including a plurality of joint units and a wire connecting the plurality of joint units to each other and configured to guide the electrode unit;
a sensor unit formed in a predetermined portion of the electrode unit and configured to measure a temperature of the tube in the body; and
a signal processor configured to convert an analog signal value measured by the sensor unit into a digital signal value and transmit the converted digital signal value to an RF energy generator.

2. The electrode apparatus of Claim 1,
wherein the electrode unit includes:
a base layer;
a plurality of electrode layers disposed on the base layer; and
a top layer disposed to overlap a part of the electrode layers, with the electrode layers interposed between the base layer and the top layer.

3. The electrode apparatus of Claim 2,
wherein the sensor unit is a thermocouple composed of a first metal and a second metal, and
the thermocouple includes a cold junction disposed on the base layer.

4. The electrode apparatus of Claim 1, further comprising:
a driving unit which is disposed inside the main body and configured to control the joint units and the wire to increase a difference in displacement therebetween in order for the electrode guide to be in a wound state to enclose the circumference of the tube in the body and control the joint units and the wire to decrease the difference in displacement therebetween in order for the electrode guide to be in a rectilinear state.

5. The electrode apparatus of Claim 4,
wherein the driving unit includes:
a rod block whose one end is connected to the joint unit and which is moved in forward and backward directions;
a wire block which is configured to support the wire and moved in the forward and backward directions; and
a variable connection unit which is configured to connect the rod block and the wire block to each other and vary a distance between the rod block and the wire block.

6. The electrode apparatus of Claim 4,
wherein the driving unit includes:
a motor unit;
a rod block which includes a rod whose one end is connected to the joint unit and is moved in the forward and backward directions by means of the motor unit; and
a wire block which is configured to support the wire and moved in the forward and backward directions in parallel to the rod block, and
a distance between the wire block and the rod block increases when the rod block moves in the forward direction and the distance between the wire block and the rod block decreases when the rod block moves in the backward direction.

7. The electrode apparatus of Claim 1,
wherein the wire is protruded from one end of the shaft with a smaller displacement than the joint units and provides a force of pulling the joint units in a direction to be wound around the tube.

8. The electrode apparatus of Claim 1,
wherein each joint unit includes:
a hinge unit which is formed on one or both sides of the joint unit in a longitudinal direction to be connected to an adjacent joint unit; and
a wire hole which is formed to allow insertion of the wire at a location spaced apart from a rotation center of the hinge unit.

9. The electrode apparatus of Claim 1,
wherein the plurality of joint units is made of an elastically deformable material and formed as one body, and
a winding support groove of which at least a part of a space is deformed to be closed by a force of the wire is formed between adjacent joint units of the electrode guide.
